Europäisches Patentamt

European Patent Office

Office européen des brevets

(11) Veröffentlichungsnummer: **0 353 524**
**A2**

(12) **EUROPÄISCHE PATENTANMELDUNG**

(21) Anmeldenummer: **89112978.5**

(22) Anmeldetag: **14.07.89**

(51) Int. Cl.⁴: **G01N 15/08 , G01M 3/16 ,**
**G01N 33/36**

(30) Priorität: **02.08.88 DE 3826275**

(43) Veröffentlichungstag der Anmeldung:
**07.02.90 Patentblatt 90/06**

(84) Benannte Vertragsstaaten:
**DE FR GB IT**

(71) Anmelder: **W.L. Gore & Associates GmbH**
**Hermann-Oberth-Strasse 22**
**D-8011 Putzbrunn(DE)**

(72) Erfinder: **Kleis, Rudolf**
**Fischerstrasse 25**
**D-8152 Feldikirchen-Westerham(DE)**
Erfinder: **Ziegler, Gerd**
**Johann-Sebastian-Bach-Strasse 1**
**D-8011 Vaterstetten(DE)**

(74) Vertreter: **Klunker . Schmitt-Nilson . Hirsch**
**Winzererstrasse 106**
**D-8000 München 40(DE)**

(54) Vorrichtung zum Testen von Bekleidungsgegenständen auf Wasserdichtigkeit.

㉗ Eine erfindungsgemäße Vorrichtung zum Testen von Bekleidungsgegenständen weist eine Trägervorrichtung auf, welcher der zu testende Bekleidungsgegenstand übergezogen werden kann, der dann mit Wasser beaufschlagt wird. Dabei ist die Trägervorrichtung im wesentlichen einem menschlichen Körper oder Körperteil nachgebildet und ist an ihrer Oberfläche mit Feuchtigkeitssensoren versehen, die mit einer Auswerteeinrichtung verbunden sind.

FIG. 1

EP 0 353 524 A2

## VORRICHTUNG ZUM TESTEN VON BEKLEIDUNGSGEGENSTÄNDEN AUF WASSERDICHTIGKEIT

Die Erfindung betrifft eine Vorrichtung zum Testen von Bekleidungsgegenständen auf Wasserdichtigkeit, mit einer Trägervorrichtung, welcher der zu testende Bekleidungsgegenstand übergezogen werden kann, wobei der im trockenen Zustand über die Trägervorrichtung gezogene Bekleidungsgegenstand mit Wasser beaufschlagt und auf der Innenseite auf Feuchtigkeit untersucht wird und die Oberfläche der Trägervorrichtung mindestens an für das Eindringen von Wasser besonders kritischen Stellen des Bekleidungsgegenstands mit Feuchtigkeitssensoren versehen ist, die mit einer Auswerteeinrichtung verbunden sind.

In jüngerer Zeit sind immer mehr Bekleidungsgegenstände wie z.B. Schuhe, Stiefel, Handschuhe, Mäntel, Jacken und Hosen, als wasserdichte Bekleidungsgegenstände hergestellt worden. Dabei ist vielfach die Außenhaut solcher Bekleidungsgegenstände selbst nicht aus wasserdichtem Material hergestellt, sondern diese Bekleidungsgegenstände sind auf der Innenseite mit einer wasserdichten, wasserdampfdurchlässigen Funktionsschicht ausgekleidet, die das Eindringen von Feuchtigkeit bis zur Innenseite oder einem Innenfutter solcher Bekleidungsgegenstände verhindern soll.

Obwohl solche Bekleidungsgegenstände an sich wasserdicht sein sollen, kommt es doch immer wieder vor, daß diese Wasserdichtigkeit nicht an allen Stellen des Bekleidungsgegenstands gegeben ist. Ursache hierfür sind meist Verarbeitungsfehler oder Wasserundichtigkeit verursachende Verarbeitungsmethoden bei der Herstellung der Bekleidungsgegenstände. Häufig werden Wasserbrücken zwischen der Außenseite der Funktionsschicht und deren Innenseite bzw. dem auf deren Innenseite befindlichen Innenfutter des Bekleidungsgegenstands durch Nahtlöcher oder Nahtfäden gebildet. Auch nicht richtig abgeschnittene und daher über Schnittenden der Funktionsschicht überstehende Fäden oder Textilteile führen häufig zu Wasserbrücken. Die Folge ist, daß sich auf der Innenseite des Bekleidungsstückes Wasser ansammelt, das dann in dem im allgemeinen vorhandenen Innenfutter weiterkriecht. Beispielsweise bei wasserdichten Schuhen, die solche Wasserbrücken aufweisen, dauert es nach dem Naßwerden der Außenseite des Schuhes nur etwa 10 Minuten, bis das Innenfutter eines solchen Schuhes feucht ist.

Zum Testen von Schuhen auf Wasserdichtigkeit hat man bisher den sogenannten PFI-Tester des Prüf- und Forschungsinstituts der Schuhindustrie verwendet. Dieser umfaßt einen Stahlleisten, der nur im Fußspitzenbereich leistenähnlich ausgebildet ist, ansonsten die Form einer schmalen Leiste hat. Im Flexbereich des Schuhes, also dort, wo sich bei der Gehbewegung etwa in Höhe der Zehenansätze eine Gehfalte bildet, ist diese Stahlleiste mit einem federbelasteten Gelenk ausgebildet. Dadurch kann man die leistenähnliche Spitze gegen die Federkraft anheben und in ihre Ruhestellung zurückkehren lassen. Zu diesem Zweck wird der zu testende, über den Stahlleisten gezogene Schuh mit seinem Absatz auf eine feststehende Stahlplatte und mit seinem Zehenbereich auf eine bewegliche Stahlplatte gestellt. Diese Stahlplatte wird in eine Bewegung gebracht, die eine horizontale Komponente und eine vertikale Komponente aufweist. Hierfür ist ein aufwendiger Mechanismus erforderlich, der in das Wasserbad eingetaucht ist, das insbesondere wegen der aus den zu testenden Schuhen auslaufenden Gerbsäure agressiv ist und den Mechanismus angreift und auf Dauer schädigt.

Hinzu kommt, daß der bekannte Stahlleisten, der bis auf die Spitze die Form einer schmalen Stahlleiste oder Stahlstange hat, nur an zwei Stellen relativ kleiner Fläche eine Berührung mit dem zu testendem Schuh hat. Aufgrund des Druckes von der Bodenplatte wandert diese Stahlleiste im Schuh. An den Auflagepunkten zwischen Stahlleiste und Schuh kann es zu einem Auf- oder Zerscheuern des Futters und damit der wasserdichten Funktionsschicht kommen. Wenn dann Feuchtigkeit im Schuh festgestellt wird, läßt sich oft nicht klar unterscheiden, ob diese aufgrund einer zerscheuerten Funktionsschicht eingedrungen ist oder aufgrund irgendwelcher system- oder herstellungsbedingter Wasserbrücken.

Bei dem bekannten Schuhtester ist der Test auf Wasserundichtigkeit durch Besichtigen und Befühlen des vom Leisten abgenommenen Schuhs durchgeführt worden. Häufig hat man in den abgenommenen Schuh Löschpapier oder ähnliches eingeführt, um eventuell eingedrungene Feuchtigkeit feststellen zu können. Diese Methode ist einerseits ungenau und erlaubt andererseits keine genaue Aussage, nach wieviel Bewegungszyklen die Feuchtigkeit aufgetreten ist. Denn es hängt rein vom Zufall ab, nach wieviel Bewegungszyklen die Bedienungsperson den Schuhtester anhält, den Schuh aus dem Wasser nimmt und das Innere des Schuhs auf eingedrungene Feuchtigkeit untersucht.

Der bekannte Schuhtester ist nur zum Testen einer ganz bestimmten Schuhgröße ausgelegt. Würde man an der Haltevorrichtung für den Schuhleisten einen kürzeren Leisten für einen kleineren Schuh anbringen, befände sich die auf- und abbewegbare Bodenplatte nicht mehr an richtiger Stelle unter dem Zehenbereich des kleineren Schuhs. Entsprechend Umgekehrtes gilt für einen größeren Schuh als er für den Schuhtester vorgesehen ist.

Auch andere wasserdichte Bekleidungsstücke wie z.B. Handschuhe, Jacken, Hosen und Mäntel, sollten auf Wasserdichtigkeit testbar sein. Bisher sind solche Bekleidungsgegenstände nicht insgesamt testbar. Man hat lediglich einen Textilausschnitt eines zu testenden Bekleidungsgegenstands mit einer Wassersäule definierter Höhe belastet und auf Wasserdichtigkeit geprüft. Wie sich ganze Kleidungsstücke im angezogenen Zustand verhalten und wo besonders kritische Stellen sind, ließ sich bisher nicht testen.

Der Erfindung liegt die Aufgabe zugrunde, eine Vorrichtung zum Testen von Bekleidungsgegenständen auf Wasserdichtigkeit verfügbar zu machen, mit welcher sich Bekleidungsgegenstände in einer Situation, die der tatsächlichen Benutzung möglichst nahe kommt, mit hoher Meßgenauigkeit, Zuverlässigkeit und Aussagekraft testen lassen.

Die Lösung dieser Erfindung besteht in einer Vorrichtung zum Testen von Bekleidungsgegenständen auf Wasserdichtigkeit, mit einer Trägervorrichtung, welcher der zu testende Bekleidungsgegenstand übergezogen werden kann, wobei der im trockenen Zustand über die Trägervorrichtung gezogene Bekleidungsgegenstand mit Wasser beaufschlagt und auf der Innenseite auf Feuchtigkeit untersucht wird und die Oberfläche der Trägervorrichtung mindestens an für das Eindringen von Wasser besonders kritischen Stellen des Bekleidungsgegenstands mit Feuchtigkeitssensoren versehen ist, die mit einer Auswerteeinrichtung verbunden sind, dadurch gekennzeichnet, daß die Trägervorrichtung im wesentlichen einem menschlichen Körper oder Körperteil nachgebildet ist und daß die Auswerteeinrichtung eine Anzeigevorrichtung speist, welche die Kontur des mit dem Bekleidungsgegenstand zu bekleidenden Körperteils angibt und an den den Sensorpositionen entsprechenden Stellen optische Anzeigeelemente aufweist, die von der Auswerteeinrichtung in einen Anzeigezustand gebracht werden, wenn der zugehörige Feuchtigkeitssensor Feuchtigkeit meldet.

Die Wasserdichtigkeit imprägnierter Stoffstücke mit Hilfe von Feuchtigkeitssensoren in Form beabstandeter elektrischer Elektroden zu messen, ist an sich aus der US-PS 20 12 762 bekannt. Dort wird zwischen einem imprägnierten Stoffstück und den beiden Elektroden ein Filterpapier angeordnet und das Stoffstück mit Wasser beaufschlagt. Wird das Stoffstück von Wasser durchdrungen, wird das Filterpapier naß und überbrückt die beabstandeten Elektroden elektrisch. In dem die Elektroden enthaltenden Stromkreis fließt dann ein Strom, der mit Hilfe eines Galvanometers gemessen wird. Mit der bekannten Vorrichtung lassen sich aber nicht Bekleidungsstücke und deren Schwachstellen, z.B. dort, wo Nähte sind, auf Wasserdichtigkeit untersuchen.

Aus JP-A2 58-10651 ist es bekannt, die Hitzebeständigkeit von Bekleidungsgegenständen dadurch zu messen, daß man sie einer mit Wärmesensoren versehenen menschlichen Puppe überzieht und dann Wärmequellen auf die Puppe richtet.

Dadurch, daß erfindungsgemäß die Oberfläche der Trägervorrichtung, bei der es sich beispielsweise um einen Schuhleisten, einen Handschuhleisten oder um eine Körperpuppe handeln kann, an verschiedenen Stellen mit Feuchtigkeitssensoren versehen ist, kann der Wasserdichtigkeitstest durchgeführt werden, ohne daß der Bekleidungsgegenstand immer wieder von der Trägervorrichtung abgenommen werden muß, um zu überprüfen, ob schon Feuchtigkeit bis ins Innere vorgedrungen ist. Da die Auswertung automatisch abläuft, während der Bekleidungsgegenstand über die Trägervorrichtung gezogen ist, kann exakt derjenige Zeitpunkt ermittelt und festgehalten werden, zu welchem Feuchtigkeit ins Innere gedrungen ist. Da das Kriterium trocken oder feucht anhand der Ausgangssignale der Feuchtigkeitssensoren entschieden wird, kann, ohne daß eine Bedienungsperson zugegen ist, der Wasserdichtigkeitstest automatisch angehalten werden, sobald ein Feuchtigkeitssensor Feuchtigkeit meldet. Man kann automatisch die Testzeitdauer und/oder die Anzahl der Bewegungszyklen messen bzw. zählen, bis ein Feuchtigkeitssensor Feuchtigkeit gemeldet hat. Nach deren Ingangsetzen kann man die Testvorrichtung völlig sich selbst überlassen. Insbesondere dann, wenn man in bevorzugter Weise eine Testvorrichtung mit mehreren Trägervorrichtungen einsetzt, beispielsweise einen Schuhtester mit mehreren Fußnachbildungen oder Fußprothesen, die je getrennt voneinander gemessen und ausgewertet werden, kann man die Testvorrichtung stunden- oder auch tagelang alleine laufen lassen und braucht nach dieser Zeit nur die Testergebnisse für die einzelnen Bekleidungsstücke abzulesen. Wenn man bedenkt, daß beispielsweise wasserdichte Schuhe in einem Schuhtester bis zum Testende einer Anzahl von Bewegungszyklen unterzogen werden, die im Bereich von einigen Hunderttausend oder gar Millionen liegt, kann man sich vorstellen, wie groß die Arbeitskrafteinsparung ist. Denn im Gegensatz dazu müßte man bei dem bekannten Schuhtester, um wenigstens mit einigermaßener Genauigkeit den Zeitpunkt feststellen zu können, zu welchem Zeitpunkt Feuchtigkeit nach innen gedrungen ist, recht häufig den Test unterbrechen und die Schuhe vom Schuhleisten abnehmen, um sie auf eingedrungene Feuchtigkeit überprüfen zu können. Es müßte also praktisch während der gesamten Testdauer mindestens eine Bedienungsperson zugegen sein.

Im Fall von Bekleidungsgegenständen wie Jakken, Mänteln und Hosen wäre es selbstverständlich sehr aufwendig, alle Bereiche des Bekleidungsgegenstands, die sich erfahrungsgemäß als besonders kritisch für das Eindringen von Feuchtigkeit erweisen, mit dem bekannten Wassersäulentest zu überprüfen. Stellen, die bis dahin als nicht kritisch erkannt worden sind und sich möglicherweise nur bei einer bestimmten Herstellungsmethode als kritisch erweisen, würden bei dem bekannten Wassersäulentest ungeprüft bleiben. Wendet man dagegen die erfindungsgemäße Testvorrichtung in Form einer mit Feuchtigkeitssensoren versehenen menschlichen Puppe an, kann man eine beliebige Anzahl von Feuchtigkeitssensoren an beliebigen Stellen der Puppe anbringen, so daß sich eine Jacke oder ein Mantel in einem einzigen Testvorgang an beliebig vielen Stellen auf das Eindringen von Feuchtigkeit untersuchen lassen.

In besonders bevorzugter Weise kann man die Puppe oder Teile davon aufblasbar machen, um ein besonders gutes Anliegen der Feuchtigkeitssensoren an der Innenseite des zu testenden Bekleidungsgegenstands sicherzustellen.

Besonders vorteilhaft ist es, wenn man die Puppe mit dem zu testenden Bekleidungsstück in eine Duschkabine stellt, die eine Vielzahl von an unterschiedlichen Positionen befindlichen Duschköpfen aufweist, so daß das Bekleidungsstück praktisch von allen Seiten gleichzeitig mit Wasser beaufschlagt werden kann.

Besonders übersichtlich und einfach handhabbar ist die Testvorrichtung, da ihr eine Anzeigeeinrichtung zugeordnet ist, welche die Form des zu testenden Bekleidungsgegenstands andeutet und mit Leuchtelementen, vorzugsweise Leuchtdioden, an solchen Stellen versehen ist, die den Positionen der einzelnen Feuchtigkeitssensoren auf der Trägervorrichtung entsprechen. Dann läßt sich mit einem Blick erkennen, an welcher Stelle der zu testende Bekleidungsgegenstand undicht ist.

Hinsichtlich weiterer vorteilhafter Ausgestaltungen wird auf die Unteransprüche verwiesen.

Die Erfindung sowie weitere Aufgabenaspekte, Vorteile und Ausgestaltungen werden nun anhand von Ausführungsformen näher erläutert. In den Zeichnungen zeigen:

Fig. 1 eine als Schuh-Trägervorrichtung verwendete Fußprothese mit auf deren Oberfläche vorgesehenen Feuchtigkeitssensoren in Seitenansicht;

Fig. 2 eine Draufsicht auf die in Fig. 1 gezeigte Fußprothese;

Fig. 3 eine schematische Darstellung eines erfindungsgemäßen Schuhtesters in Seitenansicht;

Fig. 4 eine schematische Darstellung des Schuhbewegungsmechanismus des in Fig. 3 gezeigten Schuhtesters;

Fig. 5 ein Beispiel für eine Anzeigevorrichtung eines Schuhtesters;

Fig. 6 eine schematische Darstellung einer mit Feuchtigkeitssensoren versehenen menschlichen Puppe,die in einer Duschkabine angeordnet ist;

Fig. 7 eine Draufsicht auf die in Fig. 6 gezeigte Puppe mit Darstellung der Verteilung der Duschköpfe;

Fig. 8 eine schematische Rückansicht der in Fig. 6 gezeigten Puppe mit Feuchtigkeitssensoren;

Fig. 9 eine Seitenansicht der in Fig. 8 gezeigten Puppe; und

Fig. 10 ein elektrisches Blockschaltbild einer Auswerteeinrichtung einer erfindungsgemäßen Testvorrichtung.

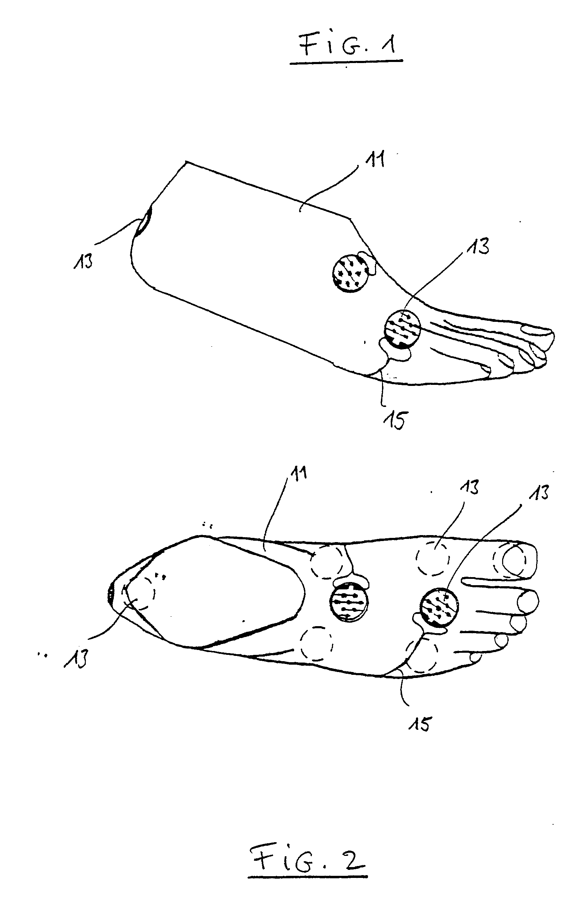

Anhand der Fig. 1 bis 5 wird zunächst eine Ausführungsform eines Schuhtesters betrachtet.

Für einen erfindungsgemäßen Schuhtester wird eine Fußnachbildung in Form einer Fußprothese 11 verwendet, die mindestens im vorderen Bereich aus elastisch flexiblem Material besteht. Dies ermöglicht es, für den auf die Fußprothese 11 gezogenen. Schuh eine Abrollbewegung durchzuführen, die der Abrollbewegung eines natürlichen Fußes beim Gehen weitgehend entsprechen kann.

An verschiedenen Stellen der Oberfläche der Fußprothese 11 sind Feuchtigkeitssensoren 13 angeordnet, die mittels elektrischer Leitungen 15 mit einer elektrischen Auswerteeinrichtung verbindbar sind.

Wie insbesondere Fig. 2 zeigt, sind die einzelnen Feuchtigkeitssensoren 13 hauptsächlich an solchen Stellen angeordnet, an welchen ein Schuh normalerweise besonderer Druck- oder Biegebelastung ausgesetzt ist, wie im Flexbereich über den Zehenansätzen, im Fersenbereich und an verschiedenen Stellen der Fußsohle. Auch unter dem freien Ende des großen Fußzehs ist ein Feuchtigkeitssensor angebracht, da der entsprechende Bereich des Schuhs beim Gehen relativ starken Belastungen ausgesetzt ist. Zusätzlich bebindet sich mindestens ein Feuchtigkeitssensor 13 im Bereich der Schuhzunge, um feststellen zu können, ob dieser für Wassereinbrüche sensible Bereich trocken bleibt.

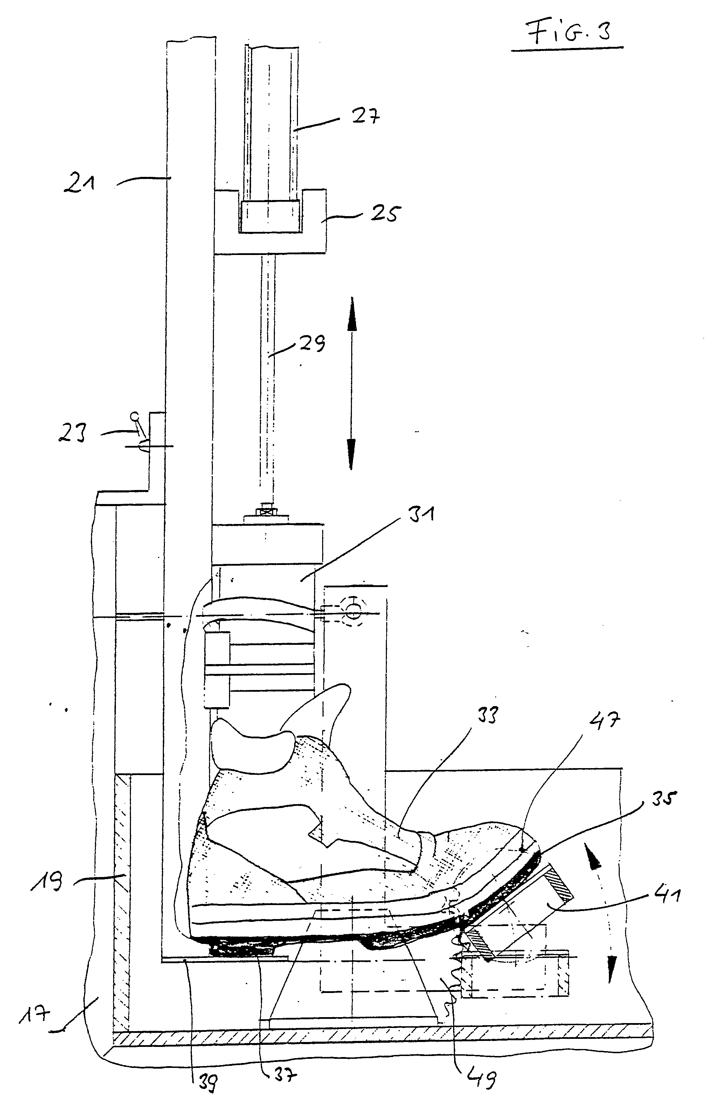

Die Testvorrichtung besteht im wesentlichen aus einer Haltevorrichtung für den zu testenden Schuh, einer Antriebseinheit für die Gehbewegung, einer Wasserwanne und einer elektronischen Steuerung mit Anzeigeeinrichtung. Der mechanische Teil der Testvorrichtung wird nun anhand von Fig. 3 näher erläutert.

An einem Gestell 17 sind eine Wasserwanne 19 und eine gegenüber dem Gestell 17 in Vertikalrichtung verstellbare Führung 21 angeordnet. Zur Höhenverstellung der Führung 21 wird ein Schraubhebel 22 gelöst und nach Einstellung der neuen Vertikalposition der Führung 21 wieder festgeschraubt. An der Führung 21 ist eine Halterung 25 für einen pneumatischen Zylinder 27 angebracht. Im pneumatischen Zylinder 27 ist eine Haltestange 29 beweglich angeordnet, an deren freiem Ende eine Haltevorrichtung 31 angeordnet ist, an deren anderem Ende die in Fig. 3 nicht sichtbare Fußprothese 11 befestigt ist. Auf die Fußprothese 11 ist ein auf Wasserdichtigkeit zu überprüfender Schuh 33 gezogen, der eine Sohle 35 und einen Absatz 37 aufweist. Der Absatz 37 steht auf einer Stellfläche 39, die mit der Führung 21 in Verbindung steht. Der Zehenbereich der Sohle 35 liegt auf einer Schwelle 41, die in einer bogenförmigen Bewegung auf- und abbewegbar ist, um für den vorderen Bereich des Schuhs 33 die Abrollbewegung beim natürlichen Gehen zu simulieren.

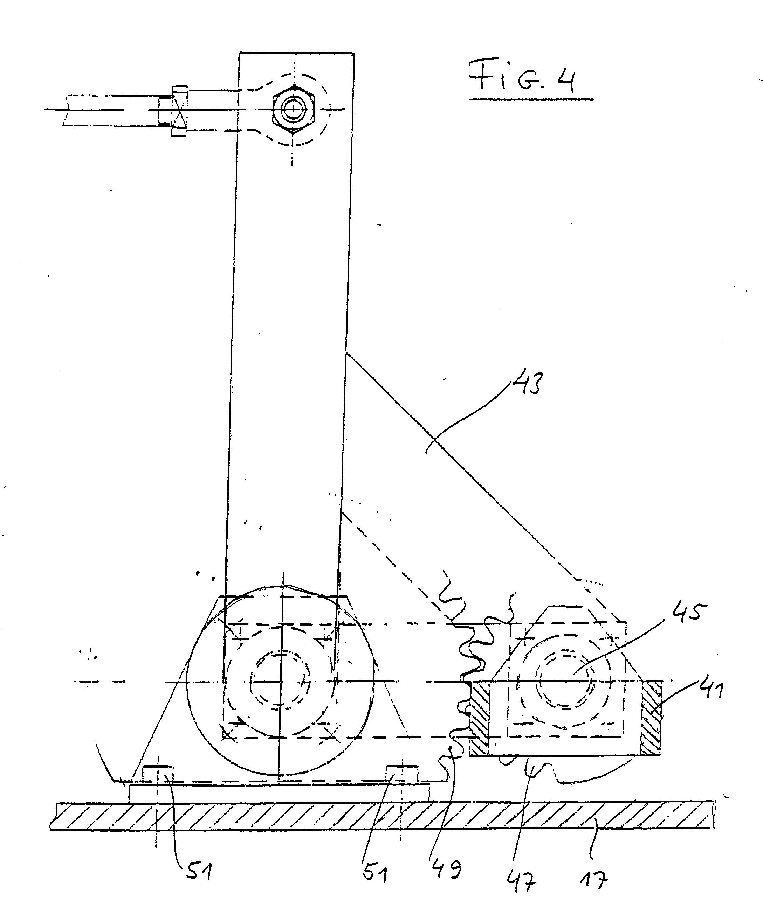

Der Bewegungsmechanismus der Schwelle 41 ist deutlicher aus Fig. 4 ersichtlich. Dieser Bewegungsmechanismus umfaßt einen Hebel 43, der über einen Kurbeltrieb von einem Motor mit einstellbarer Geschwindigkeit angehoben und abgesenkt wird. Die Schwelle 41 ist mittels einer horizontal verlaufenden Drehwelle 45 am unteren Ende des Hebels 43 verdrehbar angebracht. Auf der Drehwelle 45 befindet sich außerdem ein Zahnrad 47, das gemeinsam mit der Schwelle 41 um die Drehwelle 45 verdrehbar ist. Das Zahnrad 47 kämmt mit einem Zahnradsegment 49, das mittels Schrauben 51 am Boden des Gestells 17 befestigt ist. Bei der Auf- und Abbewegung des Hebels 43 kommt es zu einer kämmenden Abrollbewegung des Zahnrads 47 auf dem Zahnradsegment 49. Daher wird die Schwelle 41 gleichzeitig mit dem Anheben und Absenken zusammen mit dem Zahnrad 47 um die Drehwelle 45 verdreht. Die Schwelle führt daher eine bogenförmige Auf- und Abwärtsbewegung durch. Infolge der kombinierten Drehbewegung sowie Auf- und Abwärtsbewegung der Schwelle 41 führt der auf der Schwelle 41 befindliche Schuh 43 einen wirklichkeitsnahen Abrollvorgang wie beim natürlichen Laufen durch.

Durch Anordnung eines Kurbeltriebs, eines Hebels 43, eines Zahnrads 47 und eines Zahnradsegments 49 an jedem der beiden Längsenden der Schwelle 41 kann man eine lange Schwelle 41 realisieren, auf der eine Vielzahl von Schuhen nebeneinander Platz hat. Dies führt zu einer bevorzugten Ausführungsform, bei welcher längs der Schwelle 41 eine Mehrzahl voneinander unabhängiger Führungen 21 mit je einer Halterung 25, einer pneumatischen Einheit 27, 29, einer Haltevorrichtung 31, einer Fußprothese 11 und einer Stellfläche 39 angeordnet sind. Die Feuchtigkeitssensoren 13 jeder Fußprothese sind über eine elektrische Steckverbindung und ein Flachkabel an je eine Auswerteeinrichtung mit je einer Anzeigeeinheit lösbar angeschlossen. Dadurch, daß die Führungen 21 höhenverstellbar sind, kann man jede Testeinheit entsprechend der Absatzhöhe des mit dieser Testeinheit zu testenden Schuhs 33 oder Stiefels derart einstellen, daß der vordere Bereich der Sohle 35 immer in geeigneter Weise auf der Schwelle 41 aufliegt.

Zusätzlich ist jede Führung 21 (in nicht dargestellter Weise) in Schuhlängsrichtung verstellbar. Dies ermöglicht es, die Testvorrichtung auf jede beliebige Schuhlänge einzustellen, derart, daß der im Zehenbereich befindliche Teil der Sohle 35 immer in geeigneter Weise auf der Schwelle 41 aufliegt.

In einer solchen Testvorrichtung für das gleichzeitige Testen einer Mehrzahl von Schuhen ist jede Führung 21 individuell höhenverstellbar. Außerdem ist jede Stellfläche 39 individuell in Schuhlängsrichtung verstellbar. Jede einem Schuh 33 zugeordnete Testeinheit ist individuell mittels ihrer pneumatischen Einheit 27, 29 absenkbar und anhebbar. Jeder Testeinheit sind eine eigene Anzeigevorrichtung und ein eigener Bewegungszykluszähler zugeordnet. Sobald bei einer Testeinheit ein Feuchtigkeitssensor 13 Feuchtigkeit meldet, wird der betroffene Schuh 33 durch Anheben der zugehörigen Haltestange 29 mittels des pneumatischen Zylinders 27 aus dem Wasserbad herausgehoben, wobei von der Anzeigevorrichtung entnehmbar ist, welcher der Feuchtigkeitssensoren dieser Testeinheit Feuchtigkeit meldet und bei welchem Zählstand des Bewegungszykluszählers die Feuchtigkeitsmeldung erfolgt ist.

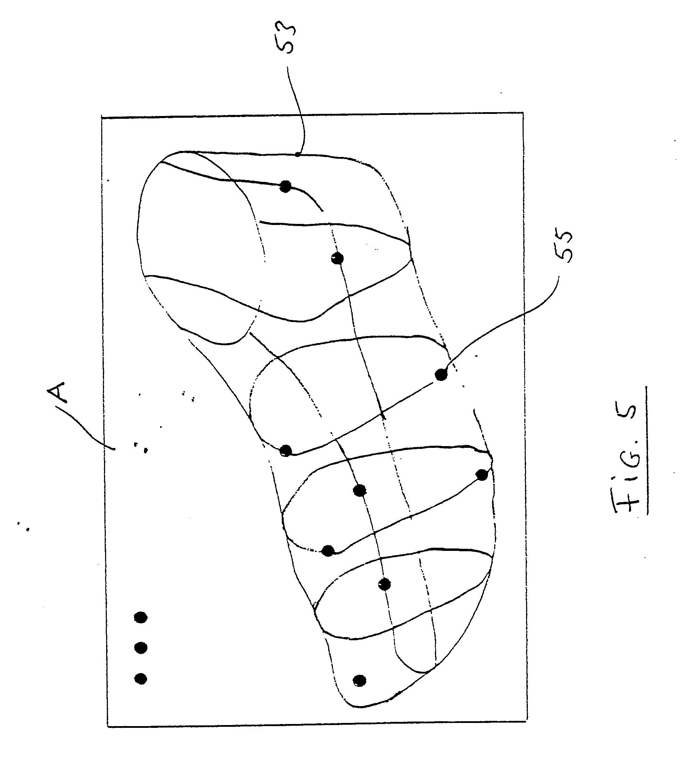

Ein Beispiel einer Anzeigevorrichtung, wie sie einer jeden Testeinheit zugeordnet ist, ist in Fig. 5 gezeigt. Demgemäß weist jede Anzeigevorrichtung eine schematische Darstellung 53 einer Fußprothese auf, wobei an den den Feuchtigkeitssensoren entsprechenden Stellen Leuchtdioden 55 angeordnet sind. Sobald ein Feuchtigkeitssensor 13 Feuchtigkeit meldet, leuchtet die zugehörige Leuchtdiode 55 auf. Es ist daher auf einen Blick feststellbar, an welcher Stelle der getestete Schuh 33 Wasser durchgelassen hat.

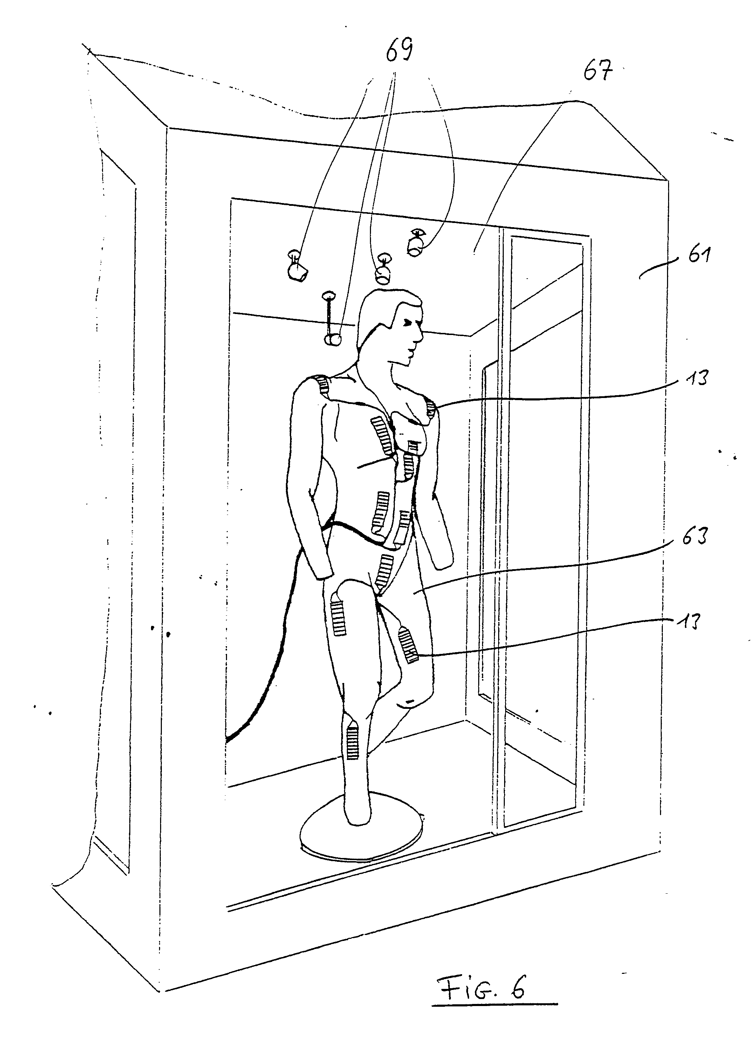

Eine bevorzugte Ausführungsform einer Testvorrichtung für Bekleidungsgegenstände wie Jakken, Hosen, Mäntel oder Overalls ist in den Fig. 6 bis 9 gezeigt. Im Inneren einer verschließbaren Duschkabine befindet sich eine Puppe 63 mit menschlicher Form und menschlicher Größe, auf deren Oberfläche eine Vielzahl von Feuchtigkeitssensoren 13 angeordnet ist. Diese befinden sich vorwiegend an für das Eindringen von Wasser besonders kritischen Stellen. Diese sind die Schulterbereiche, der Bauchbereich, wo sich häufig hinsichtlich Wasserdichtigkeit problematische Reißverschlüsse befinden, der Schrittbereich, der Oberschenkel- und Kniebereich und der Schienbeinbereich. An der Decke 67 der Duschkabine 61 befinden sich mehrere Duschköpfe 69, die sich an verschiedenen Stellen bezüglich der Puppe 63 befinden und verschiedene Wasserabstrahlrichtungen haben können.

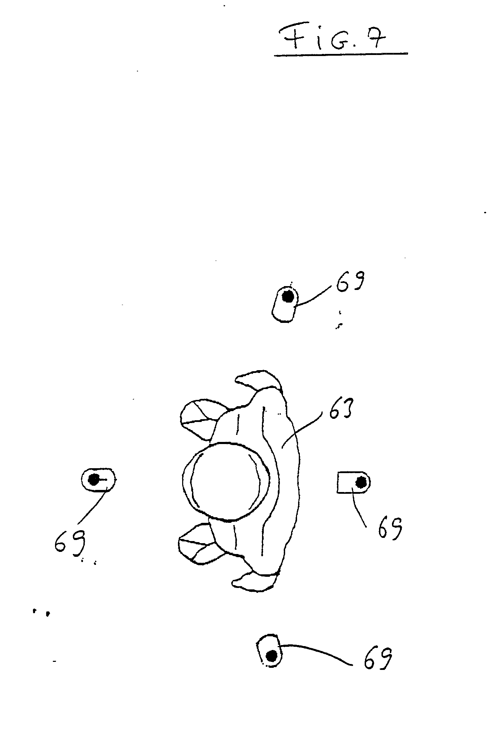

Die räumliche Verteilung der Duschköpfe 69 bezüglich der Puppe 63 ist schematisch in Fig. 7 gezeigt, die eine Draufsicht auf die Puppe 63 von oben zeigt.

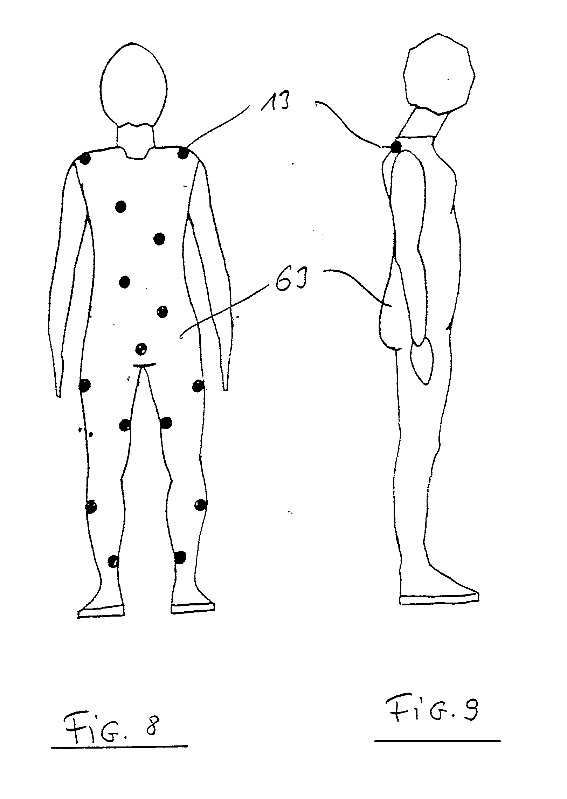

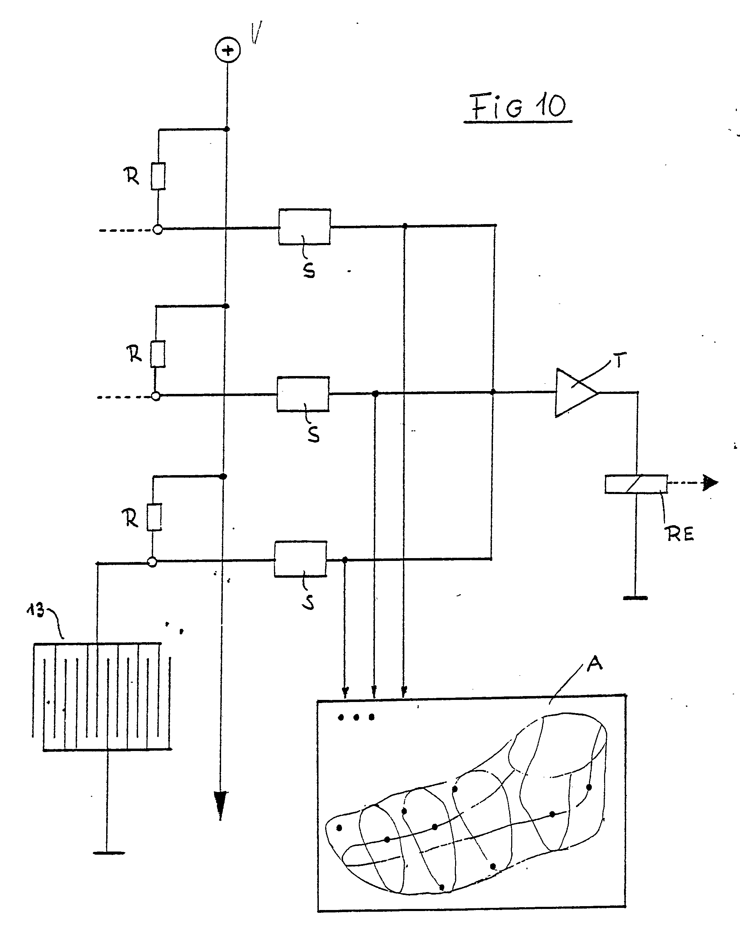

Fig. 8 zeigt eine Rückansicht der Puppe 63 mit denjenigen Stellen, an denen sich vorzugsweise Feuchtigkeitssensoren 13 befinden. Fig. 9 zeit eine Seitenansicht der Puppe mit einer bevorzugten Position für im hinteren Schulterbereich angebrachte Feuchtigkeitssensoren 13.

In einer besonders bevorzugten Ausführungsform ist die Puppe mindestens teilweise aufblasbar. Dadurch wird erreicht, daß die im aufblasbaren Bereich befindlichen Feuchtigkeitssensoren 65 mit Sicherheit zum Anliegen an die Innenseite des zu testenden Bekleidungsgegenstands gebracht werden.

Ein vereinfachtes elektrisches Blockschaltbild für eine mit mehreren Feuchtigkeitssensoren verbundene Auswerteschaltung ist in Fig. 10 gezeigt. Diese zeigt in vereinfachender Weise nur Anschlüsse für drei Feuchtigkeitssensoren 13, obwohl die Trägervorrichtung einer erfindungsgemäßen Testvorrichtung meist mehr Sensoren aufweisen wird. Dabei ist nur der untere Sensor 13 dargestellt, während für die beiden oberen Sensoren nur in gestrichelter Weise die Anschlußleitungen gezeigt sind. Die Feuchtigkeitssensoren 13 sind einerseits über je einen zugehörigen Vorwiderstand R an eine gemeinsame Spannungsquelle V und andererseits je an einen Masseanschluß angeschlossen. Die Verbindungspunkte zwischen den einzelnen Vorwiderständen und den zugehörigen Feuchtigkeitssensoren sind an je eine Schwellenwertschaltung S angeschlossen, deren Ausgänge gemeinsam mit einem Relaistreiber T verbunden sind, dessen Ausgang ein Relais RE steuert.

Bei dem in Fig. 10 gezeigten Ausführungsbeispiel weist der Feuchtigkeitssensor 13 eine Vielzahl kammartig ineinander greifender Elektroden auf, wobei benachbarte Elektroden an verschiedene Pole der Spannungsversorgungsquelle angeschlossen sind. Im völlig trockenen Zustand fließt zwischen den entgegengesetzt gepolten Elektroden kein Strom. Gelangt durch den zu testenden Bekleidungsgegenstand Feuchtigkeit auf einen Feuchtigkeitssensor 13, kommt es aufgrund der elektrischen Leitfähigkeit, die Wasser normaler Zusammensetzung aufweist, zu einem elektrischen Leiten zwischen den sich gegenüberliegenden Elektroden und damit zu einem Stromfluß durch den zugehörigen Vorwiderstand R. Dies führt wiederum zu einer Änderung des elektrischen Potentials am Eingang der zugehörigen Schwellenwertschaltung S. Übersteigt der Stromfluß durch den Feuchtigkeitssensor 13 und damit die Potentialänderung am Eingang der Schwellenwertschaltung S einen bestimmten Schwellenwert, bewirkt das Ausgangssignal des Schwellenwertschalters S über den Treiberverstärker T, daß das Relais RE anspricht. Im Fall eines Schuhtesters hat dies zur Folge, daß der Schuh, für den Wasserdurchlässigkeit gemeldet worden ist, mit Hilfe der zugehörigen pneumatischen Einheit 27, 29 aus dem Wasserbad gehoben und der diesem Schuh zugeordnete Bewegungszyklenzähler angehalten wird. Außerdem wird auf der Anzeigevorrichtung A mittels der entsprechenden Leuchtdiode angezeigt, welcher der Feuchtigkeitssensoren Feuchtigkeit meldet.

Die Feuchtigkeitssensoren 13 bestehen vorzugsweise aus zwei parallel und in sich verzweigten Kupferelektroden, die in Ätztechnik hergestellt werden. Derartige Feuchtigkeitssensoren leiden, wenn sie im nassen Zustand von Strom durchflossen werden. Daher wird bei einer bevorzugten Ausführungsform durch das Ansprechen des Relais RE auch der Stromfluß durch die Feuchtigkeitssensoren 13 unterbrochen. Damit auch anschließend noch auf der Anzeigevorrichtung A festgestellt werden kann, welcher der Feuchtigkeitssensoren 13 angesprochen hat, wird durch das Ansprechen des Relais RA auch das Anzeigemuster der Anzeigevorrichtung A festgehalten. Diese Anzeige und der Zählstand des Bewegungszyklenzählers können dann beliebig lange gehalten und angezeigt werden, beispielsweise bis zum Beginn eines neuen Testes.

Im Fall einer Testvorrichtung, wie sie in Fig. 6 gezeigt ist, wird durch Aktivieren des Relais RE vorzugsweise der Austritt von Duschwasser gestoppt. Gleichermaßen wie im Fall des Schuhtesters werden die Anzeige auf der Anzeigevorrichtung A und die von der Testzeitmeßeinrichtung ermittelte Testzeit festgehalten und angezeigt, bis diese Anzeigen nicht mehr erwünscht sind, beispielsweise weil ein neuer Test begonnen wird.

## Ansprüche

1. Vorrichtung zum Testen von Bekleidungsgegenständen (33) auf Wasserdichtigkeit, mit einer Trägervorrichtung (11; 63), welcher der zu testende Bekleidungsgegenstand (33) übergezogen werden kann, wobei der im trockenen Zustand über die Trägervorrichtung (11; 63) gezogene Bekleidungsgegenstand (33) mit Wasser beaufschlagt und auf der Innenseite auf Feuchtigkeit untersucht wird, und die Oberfläche der Trägervorrichtung (11; 63) mindestens an für das eindringen von Wasser besonders kritischen Stellen des Bekleidungsgegenstands (33) mit Feuchtigkeitssensoren (13) versehen ist, die mit einer Auswerteeinrichtung (Fig. 10) verbunden sind,
dadurch **gekennzeichnet,**

daß die Trägervorrichtung (11; 63) im wesentlichen einem menschlichen Körper oder Körperteil nachgebildet ist und daß die Auswerteeinrichtung eine Anzeigevorrichtung (A) speist, welche die Kontur des mit dem Bekleidungsgegenstand (33) zu bekleidenden Körperteils angibt und an den den Sensorpositionen entsprechenden Stellen optische Anzeigeelemente (55) aufweist, die von der Auswerteeinrichtung in einen Anzeigezustand gebracht werden, wenn der zugehörige Feuchtigkeitssensor (13) Feuchtigkeit meldet.

2. Vorrichtung nach Anspruch 1, **dadurch gekennzeichnet**, daß die Anzeigeelemente (55) durch Leuchtdioden gebildet sind.

3. Vorrichtung nach Anspruch 1 oder 2, **dadurch gekennzeichnet**, daß die Auswerteeinrichtung ein Ende der Beaufschlagung des Bekleidungsgegenstands (33) mit Wasser bewirkt, sobald ein Feuchtigkeitssensor (13) Feuchtigkeit meldet.

4. Vorrichtung nach Anspruch 1 oder 2, **dadurch gekennzeichnet**, daß die Auswerteeinrichtung einen Feuchtigkeit meldenden Feuchtigkeitssensor (13) abschaltet und der Test mit den verbleibenden Feuchtigkeitssensoren (13) weitergeführt wird.

5. Vorrichtung nach einem der Ansprüche 1 bis 4, **dadurch gekennzeichnet**, daß die Feuchtigkeitssensoren (13) je zwei elektrische Elektroden aufweisen, die einen vorbestimmten Abstand voneinander haben, der von eingedrungener Feuchtigkeit elektrisch überbrückt wird, was einen von der Auswerteeinrichtung bewertbaren Stromfluß bewirkt.

6. Vorrichtung nach einem der Ansprüche 1 bis 5, **dadurch gekennzeichnet**, daß die Auswerteeinrichtung eine Testdauermeßeinrichtung aufweist, welche die Testdauer vom Beginn der Beaufschlagung des Bekleidungsgegenstand (33) mit Wasser bis zur Feuchtigkeitsmeldung durch mindestens einen Feuchtigkeitssensor (13) feststellt, und daß die Auswerteeinrichtung die Testdauer mittels einer testdaueranzeigeeinheit zur Anzeige bringt.

7. Vorrichtung nach einem der Ansprüche 1 bis 6, **dadurch gekennzeichnet**, daß der Bekleidungsgegenstand (33) während der Beaufschlagung mit Wasser in Bewegungen gebracht wird, die natürlichen Bewegungen des Bekleidungsgegenstands (33) beim normalen Bewegen durch eine den Bekleidungsgegenstand tragende Person entsprechen, und daß die Auswerteeinrichtung die Anzahl der Bewegungszyklen vom Beginn der Beaufschlagung mit Wasser bis zur Feuchtigkeitsmeldung durch mindestens einen Feuchtigkeitssensor (13) mittels eines Bewegungszyklenzählers feststellt und mittels einer Zyklenanzeigevorrichtung zur Anzeige bringt.

8. Vorrichtung nach einem der Ansprüche 1 bis 7 zum Testen von wasserdichten Schuhen mit einer Trägervorrichtung, die mit dem über sie gezogenen Schuh (33) in Wasser getaucht wird, **dadurch gekennzeichnet**, daß die Trägervorrichtung durch eine Fußnachbildung in Form einer flexiblen Fußprothese (11) gebildet ist, auf deren Oberfläche die Feuchtigkeitssensoren (13) angeordnet sind.

9. Vorrichtung nach Anspruch 8 mit einem Bewegungsmechanismus, mittels welchem der vordere Schuhteil während des Tests zur Simulation der Gehbewegung zyklisch angehoben und in seine Ruhestelle zurückgelassen wird, wobei der Absatzbereich des Schuhs auf einer festen Unterlage gehalten wird, **dadurch gkennzeichnet**, daß der Zehenbereich des Schuhs (33) auf einer Schwelle (41) aufliegt, die eine bogenförmige Auf- und Abbewegung durchführt.

10. Vorrichtung nach Anspruch 9, **dadurch gekennzeichnet**, daß die Schwelle (41) mittels eines von einem Kurbeltrieb bewegten Hebels (43) anhebbar und absenkbar ist, daß die Schwelle (41) um eine quer zum Schuh verlaufende Längsachse (45) verdrehbar ist, und daß an der Schwelle (41) mindestens ein zusammen mit der Schwelle (41) um deren Längsachse (45) verdrehbares Zahnrad (47) oder Zahnradsegment angeordnet ist, das mit einem feststehenden Zahnrad oder Zahnradsegment (49) kämmt, dessen Mittelpunkt in Richtung von der Schuhspitze zum Schuhabsatz gesehen hinter der Schwelle (41) liegt, wodurch die Schwelle (41) beim Anhebe- und Absenkvorgang um ihre Längsachse (45) hin- und hergedreht wird.

11. Vorrichtung nach einem der Ansprüche 8 bis 10, **dadurch gekennzeichnet**, daß sich die Schwelle (41) und der zu prüfende Teil des Schuhs (33) in einem Wasserbad befinden.

12. Vorrichtung nach einem der Ansprüche 8 bis 11, **dadurch gekennzeichnet**, daß die Fußnachbildung (11) lösbar an einer Haltevorrichtung (31) befestigt ist, die zwischen einer Teststellung, in welcher sie den Schuh (33) auf eine Stellfläche (39) drückt, und einer Ruhestellung, in welcher sie den Schuh (33) aus dem Wasserbereich heraushebt, auf- und abbewegbar ist, und daß die Feuchtigkeitssensoren (13) der Fußnachbildung (11) mittels einer lösbaren Verbinderanordnung mit der Auswerteeinrichtung verbunden sind.

13. Vorrichtung nach Anspruch 12, **dadurch gekennzeichnet**, daß die Haltevorrichtung (31) pneumatisch oder hydraulisch auf- und abbewegbar ist.

14. Vorrichtung nach einem der Ansprüche 8 bis 13, **dadurch gekennzeichnet**, daß nebeneinander mehrere Haltevorrichtungen (31) für je eine Trägervorrichtung in Form einer Fußnachbildung (11) vorgesehen sind, denen eine gemeinsame Schwelle (41) zugeordnet ist.

15. Vorrichtung nach Anspruch 14, **dadurch gekennzeichnet**, daß jede Haltevorrichtung (31) unabhängig von den anderen Haltevorrichtungen (31) auf individuelle Höhe absenkbar ist und jeder Haltevorrichtung eine eigene Anzeigevorrichtung (A) und ein eigener Bewegungszyklenzähler zugeordnet sind.

16. Vorrichtung nach einem der Ansprüche 8 bis 15, **dadurch gekennzeichnet,**
daß die bzw. jede Fußnachbildung (11) in Schuhlängsrichtung relativ zur Schwelle (41) verstellbar ist.

17. Vorrichtung nach einem der Ansprüche 12 bis 16, **dadurch gekennzeichnet**, daß bei Feuchtigkeitsmeldung durch einen Feuchtigkeitssensor (13) die entsprechende Haltevorrichtung (31) zusammen mit der zugehörigen Trägervorrichtung (11) aus dem Wasser gehoben wird.

18. Vorrichtung nach Anspruch 17, **dadurch gekennzeichnet**, daß jedem Feuchtigkeitssensor (13) eine Schwellenwertschaltung (S) nachgeschaltet ist, die durch Überschreiten eines Schwellenwerts für den Stromfluß durch den zugehörigen Feuchtigkeitssensor (13) aktiviert wird,
daß die Ausgänge aller Schwellenwertschaltungen (S) gemeinsam mit einem Relais (RE) verbunden sind, das infolge der Aktivierung einer Schwellenwertschaltung (S) ein Anhalten des Bewegungszyklenzählers, ein Festhalten des Anzeigemusters der Anzeigevorrichtung (A) und ein Anheben der Haltevorrichtung (31) steuert.

19. Vorrichtung nach einem der Ansprüche 1 bis 7 zum Testen von Bekleidungsgegenständen wie Jacken, Mänteln und Hosen,
**dadurch gekennzeichnet**, daß als Trägervorrichtung eine Puppe (63) oder ein Puppelteil in menschlicher Größe vorgesehen ist, auf deren bzw. dessen Oberfläche Feuchtigkeitssensoren (13) vorgesehen sind,
und daß die Puppe (63) bzw. das Puppenteil in einer Duscheinrichtung (61) angeordnet und aus mindestens einem Duschkopf (69) mit Wasser besprühbar ist.

20. Vorrichtung nach Anspruch 19, **dadurch gekennzeichnet**, daß mindestens Teile der Puppe (63) bzw. des Puppenteils aufblasbar sind, um ein Anliegen der im aufblasbaren Bereich befindlichen Feuchtigkeitssensoren (13) an der Innenseite des Bekleidungsgegenstands sicherzustellen.

21. Vorrichtung nach Anspruch 19 oder 20, **dadurch gekennzeichnet**, daß jedem Feuchtigkeitssensor (13) eine Schwellenwertschaltung (S) nachgeschaltet ist, die durch Überschreiben eines Schwellenwerts des Stromflusses durch den zugehörigen Feuchtigkeitssensor (13) aktiviert wird,
und daß die Ausgänge aller Schwellenwertschaltungen (S) gemeinsam mit einem Relais (RE) verbunden sind, das infolge der Aktivierung einer Schwellenwertschaltung (S) ein Anhalten der Testdauermeßeinrichtung, ein Festhalten des Anzeigemusters der Anzeigevorrichtung (A) und ein Abschalten der Wasserberieselung steuert.

22. Vorrichtung nach einem der Ansprüche 1 bis 7, zum Testen von wasserdichten Handschuhen, **gekennzeichnet** durch eine Handnachbildung in Form einer flexiblen Handprothese, an deren Oberfläche Feuchtigkeitssensoren (13) angeordnet sind.

23. Vorrichtung nach einem der Ansprüche 1 bis 22, **dadurch gekennzeichnet**, daß die Trägervorrichtung (11; 63) mindestens teilweise heizbar ist.

24. Vorrichtung nach einem der Ansprüche 1 bis 23, **dadurch gekennzeichnet**, daß jedem Feuchtigkeitssensor (13) eine eigene Stromquelle (V, R) zugeordnet ist
und daß bei Feuchtigkeitsmeldung durch einen Feuchtigkeitssensor (13) die Testdauermeßeinrichtung bzw. der Bewegungszyklenzähler angehalten und die Testdauer bis zur Feuchtigkeitsmeldung bzw. der Bewegungszyklenzählerstand festgehalten wird.

25. Vorrichtung nach Anspruch 24, **dadurch gekennzeichnet**, daß die Aktivierung einer Schwellenwertschaltung (S) eine Unterbrechung des Stromflusses durch den zugehörigen Feuchtigkeitssensor (13) bewirkt.

## FiG. 1

## FiG. 2

FIG. 3

FIG. 4

A

53

55

FIG. 5

EP 0 353 524 A2

FiG. 6

FiG. 7

13

63

FIG. 8

FIG. 9

FIG 10